# FASCICULE DE BREVET EUROPEEN

(11) **EP 3 541 512 B1**
(45) Date de publication et mention de la délivrance du brevet: **30.03.2022**
(21) Numéro de dépôt: 17790854.8
(22) Date de dépôt: 20.10.2017
(51) Int. Cl.: B01L 3/00, A61B 10/00, B01L 7/04, B01L 9/06, G16H 10/40

(54) **DISPOSITIF DE SUIVI D'UN ÉCHANTILLON BIOLOGIQUE NOTAMMENT EN VUE D'UNE ANALYSE EN LABORATOIRE**
VORRICHTUNG ZUR ÜBERWACHUNG EINER BIOLOGISCHEN PROBE, INSBESONDERE IN DER VORBEREITUNG ZUR ANALYSE IN EINEM LABOR
DEVICE FOR MONITORING A BIOLOGICAL SAMPLE, PARTICULARLY IN PREPARATION FOR ANALYSIS IN A LABORATORY

(30) Priorité: 18.11.2016 FR 1670692
(43) Date de publication de la demande: 25.09.2019
(73) Titulaire: Filolab, 59113 Seclin (FR)
(72) Inventeur: SCHMITT, Roger, 59113 Seclin (FR); BINET, Philippe, 62144 Mont-Saint-Eloi (FR); FANTINI, Carlo, 62740 Fouquières-les-Lens (FR)
(74) Mandataire: Ipside
(86) Numéro de dépôt international: PCT/IB2017/056538
(87) Numéro de publication internationale: WO 2018/091990

(56) Documents cités:
- WO-A1-2013/144262
- WO-A2-02/056121
- CN-A- 102 509 210
- FR-A1- 2 825 637
- US-A1- 2006 208 881
- US-A1- 2012 256 806
- HOHBERGER CLIVE ET AL: "Applying radio-frequency identification (RFID) technology in transfusion medicine", BIOLOGICALS, vol. 40, no. 3, 10 novembre 2011 (2011-11-10), pages 209-213, XP028916839, ISSN: 1045-1056, DOI: 10.1016/J.BIOLOGICALS.2011.10.008
- ABARCA A ET AL: "Intelligent sensor for tracking and monitoring of blood temperature and hemoderivatives used for transfusions", SENSORS AND ACTUATORS A: PHYSICAL, ELSEVIER BV, NL, vol. 152, no. 2, 5 avril 2009 (2009-04-05) , pages 241-247, XP026144736, ISSN: 0924-4247, DOI: 10.1016/J.SNA.2009.03.018 [extrait le 2009-04-05]
- Fernandez José: "Nouveaux produits", Biofutur (276), 22 March 2007 (2007-03-22), pages 62-62, XP55792676, Retrieved from the Internet: URL:https://biofutur.revuesonline.com/arti cle.jsp?articleId=9602 [retrieved on 2021-04-06]

## Description

### DOMAINE TECHNIQUE DE L'INVENTION

La présente invention concerne un dispositif de suivi d'un échantillon biologique notamment en vue d'une analyse en laboratoire.

### ART ANTERIEUR DE L'INVENTION

Lorsque le professionnel de santé réalise un prélèvement au domicile du patient, d'importants problèmes de logistique et de traçabilité se posent pour préserver la qualité de l'échantillon. En effet, le professionnel de santé ne peut se rendre, après chaque prélèvement effectué, directement au laboratoire d'analyses médicales ou en pharmacie pour livrer l'échantillon en vue de son analyse et l'échantillon subit des variations de température non maîtrisés pouvant affecter sa qualité.

Dans la pratique, le professionnel de santé doit noter pour chaque prélèvement son horaire et tenir compte de l'urgence pour la livraison au laboratoire ou en pharmacie. Cette opération est fastidieuse et également insuffisante car la dégradation de l'échantillon est également due aux températures subies par l'échantillon qui ne sont pas relevées par le professionnel. ce jour, compte tenu des aléas liés au temps de déplacement, la distance jusqu'au laboratoire étant parfois importante, et surtout aux conditions de transport des échantillons sur le plan thermique, il n'est pas possible d'évaluer précisément la qualité de l'échantillon arrivé à destination au laboratoire.

Sur le plan médical cela pose un problème important puisqu'un échantillon dégradé ne permet pas une analyse satisfaisante utilisable par le médecin pour pouvoir poser un diagnostic ou modifier le traitement, par exemple.

Sur le plan réglementaire, les règlements en vigueur font peser une responsabilité de plus en plus forte sur les laboratoires en vue d'augmenter la qualité des analyses et in fine la sécurité du patient. Les laboratoires devant analyser les échantillons effectués à domicile engagent donc leur responsabilité sans maîtriser l'ensemble de la chaîne logistique. Pour limiter les risques d'erreur d'analyse, les laboratoires sont contraints d'augmenter le taux de refus d'échantillons et notamment dès qu'un doute apparaît sur le respect de la chaîne de prélèvement sur le plan des températures subies et/ou sur la durée entre le prélèvement de l'échantillon et l'analyse. Chaque refus nécessite de refaire un prélèvement ce qui est problématique à la fois sur le plan économique et sur le plan médical en ce sens que le diagnostic sur la base de l'analyse de l'échantillon prélevé est nécessairement différé.

Le document WO 2013/144262 A1 décrit un dispositif qui ne comporte ni boitier de commande avec des moyens de déclenchement manuel, ni de premiers moyens de transmission permettant l'inscription dans la puce de données relatives à l'échantillon suite à un déclenchement.

Le document US 2006/256806 A1 décrit une boîte configurée pour recevoir des échantillons.

Le document « Applying radio frequency identification (RFID) technology in transfusion medecine » de HOHBERGER CLIVE et AL publié dans la revue BIOLOGICALS volume 40, n°3 le 10 novembre 2011 (page 209-213) décrit l'utilisation de puces RFID pour les transfusions sanguines.

Le document WO 02/056121 A2 décrit une méthode de contrôle et de suivi d'échantillons.

La publication « Nouveaux produits », Biofutur (276), page 62, du 22 mars 2007, au nom de Fernandez José, divulgue un dispositif dénommé « ThermAssureRF » permettant le contrôle de température de produits à travers les emballages (sauf en métal) de sorte à garantir une traçabilité optimale de la chaine de froid et d'automatiser le processus de traitement des données. Il comporte une étiquette RFID.

### PROBLEME TECHNIQUE A RESOUDRE

Un premier but de la présente invention est de résoudre tout ou partie des problèmes techniques liés à l'art antérieur précité.

Un autre but de la présente invention est d'améliorer le contrôle de la chaîne de prélèvement, en amont de l'analyse en laboratoire.

Un autre but de la présente invention est de permettre la traçabilité des échantillons en amont de l'analyse en laboratoire.

Un autre but de la présente invention est de proposer un système de suivi d'un échantillon biologique permettant d'améliorer l'analyse biologique des échantillons et de diminuer le taux de refus d'échantillons.

Un autre but de la présente invention est de proposer un système d'enregistrement fiable et aisé pour le professionnel de santé assurant le prélèvement.

### BREVE DESCRIPTION DE L'INVENTION

La présente invention est définie par les revendications et concerne un dispositif de suivi d'un échantillon biologique notamment en vue d'une analyse en laboratoire. De manière caractéristique selon l'invention, il comprend :
- au moins un réceptacle pour la réception d'au moins un échantillon biologique, ledit réceptacle étant associé à une puce inscriptible,
- un boîtier de commande destiné à être couplé à un capteur de température, le boîtier de commande comprenant des moyens de déclenchement manuel et des premiers moyens de transmission permettant l'inscription dans la puce de données relatives à l'échantillon suite à un déclenchement, ces données relatives à l'échantillon comprenant au moins la date et l'heure du prélèvement de l'échantillon biologique, et notamment la température ambiante.
- un container pour le stockage et le transport dudit au moins un réceptacle, comportant un capteur de température, des seconds moyens de transmission permettant l'inscription, dans la puce associée à chaque réceptacle, de mesures des températures à intervalles de temps programmables,
- une unité centrale avec des moyens de traitement des données aptes à contrôler la qualité d'un échantillon d'un réceptacle en fonction des données de températures et de temps inscrites dans la puce associée. Selon un aspect avantageux de l'invention le dispositif comprend en outre un containeur central, sous forme de borne, constituant un point de dépôt pour les réceptacles, ledit containeur comportant des moyens de contrôle d'accès.

Selon un aspect avantageux de l'invention les premiers moyens de transmission permettent également l'inscription sur ladite puce d'au moins un paramètre choisi parmi l'identifiant du patient/du professionnel, le caractère urgent dudit échantillon, et le type d'échantillon.

Selon un aspect avantageux de l'invention les moyens de traitement des données sont au moins partiellement déportés dans le containeur.

Selon un aspect avantageux de l'invention le container comprend des moyens d'alerte, visuels ou sonore en cas de dépassement d'un seuil de température.

Selon un aspect avantageux de l'invention lesdits moyens de traitement desdites données sont aptes à déterminer au moins la température maximale mesurée dans ledit container et à déterminer au moins la durée de transport du ou des échantillons de chaque réceptacle.

Selon un aspect avantageux de l'invention lesdits moyens de traitement comportent une base de données et un lecteur de puce permettant de lier les données de la puce et/ou de chaque échantillon avec les données du patient.

Selon un aspect avantageux de l'invention, les moyens de traitement comportent une base de données permettant d'attribuer un niveau de qualité d'un échantillon en fonction des données de la puce et du type d'analyse à réaliser sur cet échantillon.

Selon un aspect avantageux de l'invention la puce est une puce radio fréquence réinscriptible notamment de type RFID ou NFC.

Selon un aspect avantageux de l'invention le container est isotherme et/ou comprend des moyens de régulation de la température.

### BREVE DESCRIPTION DES FIGURES

La présente invention, ses caractéristiques et les différents avantages qu'elle procure apparaîtront mieux à la lecture de la description qui suit d'un mode de réalisation particulier du dispositif de l'invention, présenté à titre d'exemple non limitatif et qui fait référence aux dessins annexés sur lesquels :
- la fig. 1 représente une première partie du dispositif de l'invention ;
- la fig. 2 représente un premier exemple de réalisation du dispositif conforme à l'invention ;
- la fig. 3 représente un exemple d'informations pouvant être affichées par l'écran de l'unité centrale ;
- la fig.4 représente un second exemple de réalisation du dispositif conforme à l'invention.

### DEFINITIONS

Le terme « échantillon » désigne tout matériel biologique provenant de l'organisme d'un être vivant ; il peut s'agir d'un fluide ou d'un morceau de tissu. Le fluide peut être un fluide excrété naturellement par l'organisme ou en être prélevé. L'échantillon peut être, par exemple, de l'urine, du sang, un échantillon de tissu obtenu par biopsie, des selles. Le terme « échantillon » peut englober à la fois le matériel biologique et le premier container contenant ce matériel biologique.

### DESCRIPTION DETAILLEE DE L'INVENTION

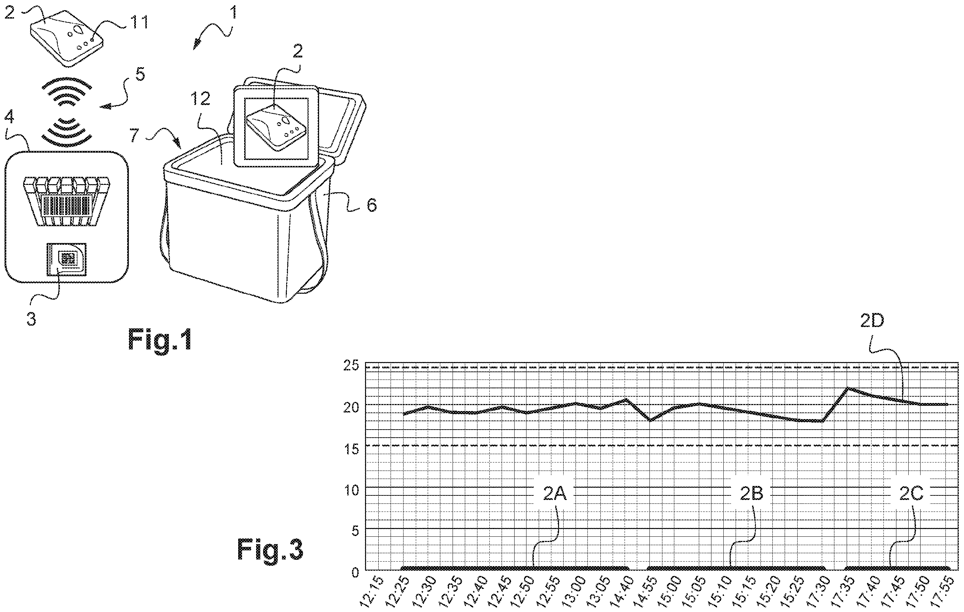

En référence à la Fig. 1, un mode de réalisation particulier du dispositif de l'invention va maintenant être décrit. Selon ce mode de réalisation, le dispositif 1 comporte un boîtier de commande 2, une puce réinscriptible à distance 3 solidaire d'un réceptacle 4, ce dernier permettant la réception des échantillons biologiques, le boîtier de commande 2 comportant des premiers moyens de transmission 5. Ces échantillons se présentent de manière avantageuse sous la forme d'une pluralité de tubes renfermant du sang tandis que le réceptacle 4 se présente sous la forme d'une pochette.

Le dispositif 1 comprend également un container 6 avantageusement sous forme d'un sac isotherme, ce container 6 est équipé d'un capteur de température 12 et de seconds moyens de transmission 7, disposés dans ledit containeur 6.

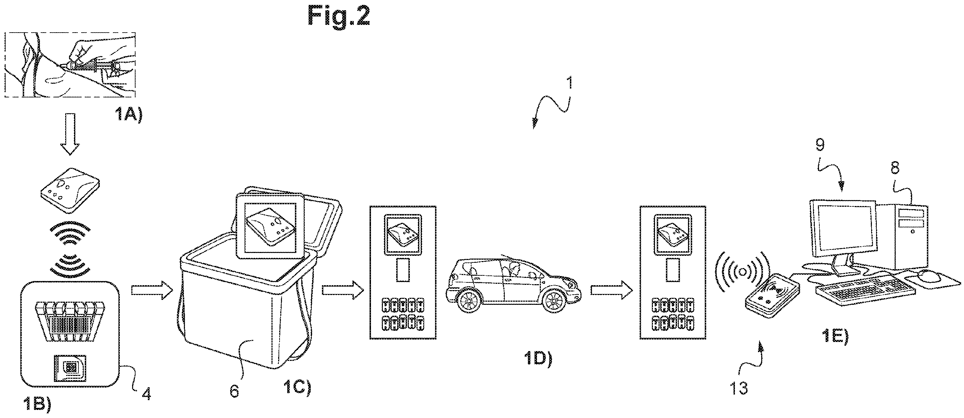

En se reportant aux figures 2 et 3 on voit que le dispositif 1 comprend en outre une unité centrale 8 avec notamment des moyens de traitement 9, ces moyens de traitement 9 pouvant être au moins partiellement déportés dans le container 6 ou dans le boîtier de commande 2 permettant de réaliser certaines opérations de traitement en mode local ou à distance. Cette unité centrale 8 est, de préférence, installée dans le laboratoire d'analyses médicales.

Le boîtier de commande 2 comprend des moyens de déclenchement manuel 10 avec un bouton poussoir 11, l'appui sur le bouton 11 permet d'activer l'inscription de la date et de l'heure sur la puce 3.

Dans une variante non représentée de ce mode de réalisation particulier, le boîtier de commande 2 comprend un second bouton poussoir, l'appui sur ce second bouton inscrivant sur la puce 3 le fait que l'échantillon doit être analysé en urgence.

Lorsque le dispositif revendiqué est en service, le boîtier de commande 2 est couplé à un capteur de température qui mesure la température ambiante. De manière générale, si l'on considère que l'échantillon prélevé va être très rapidement introduit dans le container, cette mesure de la température ambiante n'est pas nécessaire. Néanmoins, l'enregistrement de la température ambiante lors du prélèvement peut également s'avérer utile pour l'analyse biologique ultérieure de l'échantillon.

Dans le contexte de l'invention revendiquée, le boîtier de commande 2 est donc destiné à être couplé à un capteur de température qui mesure la température ambiante et les moyens de déclenchement manuel 10 activent les premiers moyens de transmission 5 de manière à inscrire sur la puce 3, l'heure du prélèvement, et également d'autres types d'information, notamment la température ambiante mesurée par le capteur de température. Peuvent également être inscrites sur la puce 3 la date ou encore le nom ou le code du professionnel ayant réalisé le prélèvement lorsque le boîtier de commande 2 est lié à ce dernier.

Dans une variante de réalisation, le boîtier 2 comporte des moyens d'inscription pour inscrire également sur ladite puce 3 au moins un paramètre chois parmi le nom de la personne ayant effectué ledit prélèvement, le caractère urgent dudit échantillon, le type d'échantillon ou encore les conditions de prélèvement. Ces moyens d'inscription peuvent constituer en un clavier ou encore une commande vocale. À cette fin, le boîtier de commande 2 est réalisé de manière avantageuse par un téléphone portable équipé de la technologie RFID ou NFC ou tout autre dispositif électronique radiofréquence équipé d'un clavier ou d'une commande vocale.

Il est ainsi possible d'inscrire également dans la puce 3 des informations particulières telles que le respect des conditions de prélèvement par le patient, et par exemple indiquer le respect d'un jeun, d'une prise de médicament antérieure, d'une bonne hydratation ou encore des conditions spécifiques à son traitement ou encore indiquer l'état du patient et notamment indiquer des états fiévreux ou grippaux pouvant modifier l'analyse à réaliser, ces données pouvant être très importantes lors de l'analyse.

En référence à la Fig. 2, un mode de fonctionnement d'un premier mode de réalisation particulier du dispositif de suivi 1 selon l'invention va maintenant être décrit.

L'étape 1A est le prélèvement de l'échantillon biologique. Dans le cas présent, il s'agit d'une prise de sang. Les tubes remplis de sang formant l'échantillon biologique sont enfermés dans une pochette, faisant office de réceptacle 4, sur laquelle est collée la puce 3.

Lors de l'étape 1B, l'infirmier déclenche à l'aide du boîtier de commande 2 l'inscription sur la puce 3 de la date du prélèvement et de l'heure du prélèvement. Il peut également, à titre éventuel inscrire également le fait que l'échantillon doit être analysé de manière urgente et/ou le type d'échantillon (urine, sang, biopsie ou autre). Il peut également inscrire la mesure de la température ambiante si le boîtier de commande 2 est couplé à un capteur de température.

Lors de l'étape 1C, l'échantillon 2 muni de la puce 3 est inséré dans le sac isotherme 6 faisant office de container 6. Les seconds moyens de transmission 7 sont paramétrés pour inscrire à intervalles prédéterminés, par exemple, toutes les 5 minutes, la température mesurée dans le sac isotherme 5 par le capteur de température 12.

Dans le cas présent, les seconds moyens de transmission 7 inscrivent sur la puce 3, toutes les 5 minutes l'heure et la température dans le sac isotherme 6.

Lors de l'étape 1D, le professionnel de santé transporte l'échantillon 2 contenu dans le sac isotherme 6, soit directement vers le laboratoire d'analyse, soit en passant chez d'autres patients pour effectuer d'autres prélèvements. Pendant cette étape les seconds moyens de transmission 7 inscrivent sur la puce 3 les mêmes informations, c'est-à-dire l'heure et la température mesurée à ladite heure par le capteur de température 12. Si le sac 6 contient plusieurs échantillons comprenant chacun une puce 3, l'inscription se fait sur chacune des puces 3.

L'étape 1E est la remise au laboratoire de l'échantillon 2. Chaque réceptacle 6 est pris en charge dans le laboratoire.

L'unité centrale 8 est équipée de moyens de lecture 13, tels qu'un lecteur de puce, adaptés des informations contenues dans ladite puce 3. Les moyens de traitement 9 permettent de traiter les données de la puce 3, avantageusement ces moyens de traitement 9 comportent en outre un afficheur permettant d'afficher la courbe de température de l'échantillon en fonction du temps.

En se reportant à la figure 3 on voit représenté un exemple de représentation de cette courbe pour une puce 3.

Les moyens de traitement 9 en plus de traiter les données de la puce 3 pour les afficher peuvent également attribuer un niveau de qualité à un échantillon en fonction des données de la puce et du type d'analyse à réaliser sur cet échantillon, à cette fin les moyens de traitement comportent une base de données. À titre d'exemple, la base de données comportera différents seuils maxima de température pour la validité de l'échantillon en fonction du type d'analyse et par exemple en fonction d'une analyse d'urine ou d'une analyse sanguine. A titre d'exemple la base de données comportera de seuils maximums de temps de transport avant analyse en fonction du type d'analyse à réaliser.

Selon un aspect avantageux de l'invention les moyens de traitement 9 et la base de données permettent de lier les données de la puce et/ou de chaque échantillon avec les données du patient.

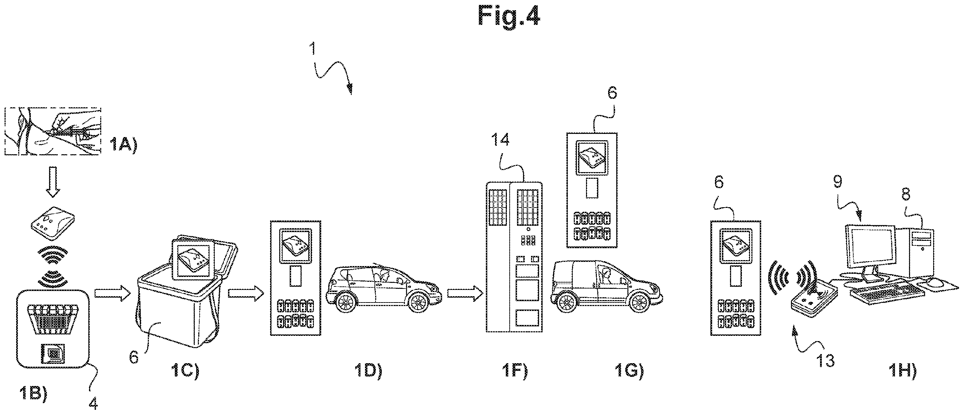

En se reportant à la figure 4 on voit représenté une variante de réalisation dans laquelle le dispositif de suivi 1 comporte un en outre un containeur central 14, sous forme de borne. Cette borne 14 constitue un point de dépôt pour les réceptacles 4 et comporte des moyens de contrôle d'accès. Le contrôle d'accès permet à la fois de contrôler le dépôt par les professionnels ayant réalisé le prélèvement et également permettre la récupération des échantillons par un personne accrédité par le laboratoire.

L'intérêt d'une telle borne et de créer un point de dépôt pour les professionnels permettant de réduire la longueur des déplacements en n'obligeant pas ces derniers à se rendre à chaque fois jusqu'au laboratoire.

De manière avantageuse, ce container central 14 comprend en plus des caractéristiques techniques des containers 6, telles que les moyens de traitement déportés, les seconds moyens de transmission et un capteur de température, différentes caractéristiques additionnelles et notamment des moyens d'accès sécurisés, des moyens de fonctionnement autonomes en énergie ou encore des moyens d'alerte. Notons que ces moyens d'alerte peuvent également être présents dans une variante de réalisation dans les containeurs 6.

En se reportant à la fig. 4 on voit que le dispositif de suivi dans ce second mode de réalisation est très proche de celui de la figure 2, mis à part le fait que les échantillons ne sont pas remis par le personnel de santé ayant prélevé l'échantillon au laboratoire mais insérés par ce dernier dans le containeur central 14. L'étape 1F est donc différente de celle concernant l'utilisation du premier mode de réalisation du dispositif de suivi 1 de l'invention.

Lorsque les échantillons 2 sont introduits dans le containeur central 14, ce dernier inscrit, via notamment les seconds moyens de transmission, la température et l'heure sur les puces 3 de chacun des échantillons (étape 1G). De la même manière, la température est mesurée, par exemple toutes les 5 minutes

Lors de l'étape 1H, le personnel du laboratoire récupère les échantillons contenus dans le containeur central 14 et les place dans un containeur 6 (pouvant également être le coffre isotherme d'un véhicule) qui inscrit à son tour les données, notamment de température, dans les puces 3. Il transporte alors ce bac vers le laboratoire. L'étape 1I est l'étape de traitement au laboratoire. Elle est identique à celle décrite en référence à la Fig. 2.

La Fig. 3 représente un exemple d'affichage sur l'écran de l'unité centrale déportée 9 des données inscrites dans la puce 3 et traitées par les moyens de traitement 9. La plage de temps 2A correspond à la durée de stockage de l'échantillon dans le containeur 6 sous forme de sac isotherme 6 ; la plage de temps 2B correspond à la durée de stockage de l'échantillon dans le containeur central 14. La plage de temps 2C correspond à la durée de stockage dans le containeur 6 sous forme de bac isotherme, lors du transport des échantillons dans le containeur 6 du laboratoire.

La courbe 2D représente la température en fonction du temps, mesurée par les différents capteurs de température. L'unité centrale déportée 8 peut également afficher la température maximale mesurée par les capteurs, la durée totale de stockage, la température minimale enregistrée sur la puce, le nom de l'infirmer et/ou du patient si ceux-ci ont été enregistrés sur la puce ou encore la référence des containeurs 6.

Le dispositif de suivi 1 tel que décrit permet ainsi une traçabilité complète des températures de stockage des réceptacles 4 contenant les échantillons depuis le prélèvement de ces échantillons et jusqu'à leurs analyses.

Bien entendu, d'autres caractéristiques de l'invention auraient également pu être envisagées sans pour autant sortir du cadre de l'invention définie par les revendications ci-après.

À titre d'exemple, selon une variante de réalisation, le container comporte des moyens d'alerte, visuels ou sonore qui sont couplés au deuxième capteur de température et qui permettent d'alerter le professionnel de santé lorsque la température mesurée dans le deuxième container est supérieure à une valeur donnée préenregistrée.

Selon une variante de réalisation, le container comporte, en outre, des moyens pour inscrire sur ladite puce un élément indiquant l'entrée dudit échantillon dans ladite zone de stockage. Il est ainsi plus aisé de déterminer le début du stockage de l'échantillon dans le dispositif de collecte. Dans le cas du container central ou borne, ces moyens peuvent, par exemple, coopérer avec le mécanisme d'ouverture d'accès à l'intérieur de la zone de stockage.

## Revendications

1. Dispositif (1) de suivi d'un échantillon biologique, notamment en vue d'une analyse en laboratoire, comprenant:
- au moins un réceptacle (4) pour la réception d'au moins un échantillon biologique, ledit réceptacle (4) étant associé à une puce inscriptible (3),
- un container (6) pour le stockage et le transport dudit au moins un réceptacle, comportant un capteur de température (12), des seconds moyens de transmission (7) permettant l'inscription, dans la puce (3) associée à chaque réceptacle (4), de mesures des températures à intervalles de temps programmables,
- une unité centrale (8) avec des moyens de traitement (9) des données aptes à contrôler la qualité d'un échantillon d'un réceptacle (4) en fonction des données de températures et de temps inscrites dans la puce (3) associée, **caractérisé en ce que**
le dispositif comprend en outre un boîtier de commande (2) destiné à être couplé à un capteur de température, le boîtier de commande (2) comprenant des moyens de déclenchement manuel (10) et des premiers moyens de transmission (5) permettant l'inscription dans la puce de données relatives à l'échantillon suite à un déclenchement, ces données relatives à l'échantillon comprenant au moins la date et l'heure de prélèvement de l'échantillon biologique et notamment la température ambiante mesurée par le capteur de température.

2. Dispositif (1) de suivi d'un échantillon biologique selon la revendication 1, **caractérisé en ce que** le dispositif (1) comprend en outre un containeur central (14), sous forme de borne, constituant un point de dépôt pour les réceptacles (4), ledit containeur (14) comportant des moyens de contrôle d'accès.

3. Dispositif (1) de suivi d'un échantillon biologique selon l'une quelconque des revendications 1 et 2, **caractérisé en ce que** les premiers moyens de transmission (5) permettent également l'inscription sur ladite puce (3) d'au moins un paramètre choisi parmi l'identifiant du patient/du professionnel, le caractère urgent dudit échantillon, et le type d'échantillon.

4. Dispositif (1) de suivi d'un échantillon biologique selon l'une quelconque des revendications précédentes, **caractérisé en ce que** les moyens de traitement (9) des données sont au moins partiellement déportés dans le containeur (6, 14).

5. Dispositif (1) de suivi d'un échantillon biologique selon la revendication 4, **caractérisé en ce que** le container (6, 14) comprend des moyens d'alerte, visuels ou sonore en cas de dépassement d'un seuil de température.

6. Dispositif (1) de suivi d'un échantillon biologique selon l'une des revendications précédentes, **caractérisé en ce que** lesdits moyens de traitement (9) desdites données sont aptes à déterminer au moins la température maximale mesurée dans ledit container (6, 14) et à déterminer au moins la durée de transport du ou des échantillons de chaque réceptacle (4).

7. Dispositif (1) de suivi d'un échantillon biologique selon l'une des revendications précédentes, **caractérisé en ce que** lesdits moyens de traitement (9) comportent une base de données et un lecteur de puce permettant de lier les données de la puce (3) et/ou de chaque échantillon avec les données du patient.

8. Dispositif (1) de suivi d'un échantillon biologique selon l'une quelconque des revendications précédentes dans lequel les moyens de traitement (9) comportent une base de données permettant d'attribuer un niveau de qualité d'un échantillon en fonction des données de la puce (3) et du type d'analyse à réaliser sur cet échantillon.

9. Dispositif (1) de suivi d'un échantillon biologique selon l'une quelconque des revendications précédentes dans lequel la puce (3) est une puce réinscriptible notamment de type RFID ou NFC.

10. Dispositif (1) de suivi d'un échantillon biologique selon l'une quelconque des revendications précédentes dans lequel le container (6, 14) est isotherme et/ou comprend des moyens de régulation de la température.

## Patentansprüche

1. Vorrichtung (1) zur Überwachung einer biologischen Probe, insbesondere im Hinblick auf eine Laboranalyse, umfassend:
- mindestens ein Behältnis (4) für die Aufnahme mindestens einer biologischen Probe, wobei das Behältnis (4) einem beschreibbaren Chip (3) zugeordnet ist,
- einen Behälter (6) für die Lagerung und den Transport des mindestens einen Behältnisses, der einen Temperatursensor (12), zweite Übertragungsmittel (7), die das Beschreiben des jedem Behältnis (4) zugeordneten Chips (3) mit Temperaturmessungen in programmierbaren Zeitintervallen gestatten, aufweist,
- eine Zentraleinheit (8) mit Verarbeitungsmitteln (9) der Daten, die imstande sind, die Qualität einer Probe eines Behältnisses (4) in Abhängigkeit der Temperatur- und Zeitdaten zu kontrollieren, mit denen der zugeordnete Chip (3) beschrieben ist, **dadurch gekennzeichnet, dass** die Vorrichtung ferner einen Steuerkasten (2) umfasst, der bestimmt ist, mit einem Temperatursensor verbunden zu sein, wobei der Steuerkasten (2) manuelle Auslösemittel (10) und erste Übertragungsmittel (5) umfasst, die das Beschreiben des Chips mit Daten gestatten, die sich auf die Probe infolge einer Auslösung beziehen, wobei sich diese Daten, die sich auf die Probe beziehen, mindestens das Datum und die Uhrzeit der Entnahme der biologischen Probe und insbesondere die von dem Temperatursensor gemessene Umgebungstemperatur umfassen.

2. Vorrichtung (1) zur Überwachung einer biologischen Probe nach Anspruch 1, **dadurch gekennzeichnet, dass** die Vorrichtung (1) ferner einen zentralen Behälter (14) in Form einer Säule umfasst, die einen Lagerplatz für die Behältnisse (4) bildet, wobei der Behälter (14) Zugangskontrollmittel aufweist.

3. Vorrichtung (1) zur Überwachung einer biologischen Probe nach einem der Ansprüche 1 und 2, **dadurch gekennzeichnet, dass** die ersten Übertragungsmittel (5) ebenfalls das Beschreiben des Chips (3) mit mindestens einem Parameter gestatten, der aus der Kennung des Patienten/des Fachmanns, der Dringlichkeit der Probe und dem Typ der Probe ausgewählt ist.

4. Vorrichtung (1) zur Überwachung einer biologischen Probe nach einem der vorangehenden Ansprüche, **dadurch gekennzeichnet, dass** die Verarbeitungsmittel (9) der Daten mindestens teilweise in den Behälter (6, 14) versetzt sind.

5. Vorrichtung (1) zur Überwachung einer biologischen Probe nach Anspruch 4, **dadurch gekennzeichnet, dass** der Behälter (6, 14) visuelle oder akustische Alarmmittel für den Fall der Überschreitung einer Temperaturschwelle umfasst.

6. Vorrichtung (1) zur Überwachung einer biologischen Probe nach einem der vorangehenden Ansprüche, **dadurch gekennzeichnet, dass** die Verarbeitungsmittel (9) der Daten imstande sind, mindestens die im Behälter (6, 14) gemessene Maximaltemperatur zu bestimmen und mindestens die Transportdauer der Probe(n) jedes Behältnisses (4) zu bestimmen.

7. Vorrichtung (1) zur Überwachung einer biologischen Probe nach einem der vorangehenden Ansprüche, **dadurch gekennzeichnet, dass** die Verarbeitungsmittel (9) eine Datenbank und ein Chiplesegerät aufweisen, das erlaubt, die Daten des Chips (3) und/oder jeder Probe mit den Daten des Patienten zu verbinden.

8. Vorrichtung (1) zur Überwachung einer biologischen Probe nach einem der vorangehenden Ansprüche, wobei die Verarbeitungsmittel (9) eine Datenbank aufweisen, die gestattet, ein Qualitätsniveau einer Probe in Abhängigkeit von den Daten des Chips (3) und des für diese Probe durchzuführenden Analysetyps zuzuweisen.

9. Vorrichtung (1) zur Überwachung einer biologischen Probe nach einem der vorangehenden Ansprüche, wobei der Chip (3) ein wiederbeschreibbarer Chip insbesondere vom Typ RFID oder NFC ist.

10. Vorrichtung (1) zur Überwachung einer biologischen Probe nach einem der vorangehenden Ansprüche, wobei der Behälter (6, 14) isotherm ist und/oder Mittel zum Einstellen der Temperatur umfasst.

## Claims

1. A device (1) for monitoring a biological sample, particularly in preparation for analysis in a laboratory, comprising:
- at least one receptacle (4) for receiving at least one biological sample, said receptacle (4) being associated with a recordable chip (3),
- a container (6) for storing and transporting said at least one receptacle, including a temperature sensor (12), second transmission means (7) allowing temperature measurements to be recorded onto the chip (3) associated with each receptacle (4), at programmable time intervals,
- a central unit (8) with data processing means (9) capable of controlling the quality of a sample of a receptacle (4) according to the temperature and time data recorded on the associated chip (3), **characterised in that** the device further comprises a control housing (2) intended to be coupled to a temperature sensor, the control housing (2) comprising manual triggering means (10) and first transmission means (5) allowing the recording of sample-related data onto the chip after a triggering process has been carried out, these sample-related data comprising at least the date and time of sampling of the biological sample and particularly the measured ambient temperature by the temperature sensor.

2. The device (1) for monitoring a biological sample according to claim 1, **characterised in that** the device (1) further comprises a central container (14), in the form of a terminal, constituting a deposit point for the receptacles (4), said container (14) including access control means.

3. The device (1) for monitoring a biological sample according to any one of claims 1 and 2, **characterised in that** the first transmission means (5) also allow at least one parameter selected from the identifier of the patient/professional, the urgency of said sample, and the type of sample, to be recorded onto said chip (3).

4. The device (1) for monitoring a biological sample according to any one of the preceding claims, **characterised in that** the data processing means (9) are at least partially remote in the container (6, 14).

5. The device (1) for monitoring a biological sample according to claim 4, **characterised in that** the container (6, 14) comprises visual or audible warning means in the event of exceeding a temperature threshold.

6. The device (1) for monitoring a biological sample according to one of the preceding claims, **characterised in that** said means (9) for processing said data are able to determine at least the maximum temperature measured in said container (6, 14) and to determine at least the transport duration of the sample (s) of each receptacle (4) .

7. The device (1) for monitoring a biological sample according to one of the preceding claims, **characterised in that** said processing means (9) include a database and a chip reader allowing to link the data of the chip (3) and/or each sample with the patient data.

8. The device (1) for monitoring a biological sample according to any one of the preceding claims, wherein the processing means (9) include a database allowing to assign a quality level of a sample according to the chip data (3) and the type of analysis to be performed on this sample.

9. The device (1) for monitoring a biological sample according to any one of the preceding claims, wherein the chip (3) is a re-recordable chip, particularly of the RFID or NFC type.

10. The device (1) for monitoring a biological sample according to any one of the preceding claims, wherein the container (6, 14) is isotherm and/or comprises means for regulating the temperature.
